(19) Europäisches Patentamt
European Patent Office
Office européen des brevets

(11) Publication number: 0 023 097
B1

(12) # EUROPEAN PATENT SPECIFICATION

(45) Date of publication of patent specification: 01.08.84

(51) Int. Cl.³: C 07 D 205/08, C 07 D 405/06, C 07 D 409/12

(21) Application number: 80302197.1

(22) Date of filing: 30.06.80

(54) De-arylmethylation of N-mono- or N-diarylmethyl-beta-lactams; novel azetizinones having anti-bacterial activity.

(30) Priority: 28.06.79 JP 82411/79
05.07.79 JP 85610/79

(43) Date of publication of application:
28.01.81 Bulletin 81/4

(45) Publication of the grant of the patent:
01.08.84 Bulletin 84/31

(84) Designated Contracting States:
BE CH DE FR GB IT LI

(56) References cited:
US - A - 3 943 123
US - A - 4 072 674
US - A - 4 166 816

TETRAHEDRON LETTERS, No. 30, July 1979 Oxford A.K. BOSE et al. "Non-Hazardous Synthesis of Isocephosporin Intermediates Via alpha Vinylamino-beta-Lactams" pages 2771 to 2774

(73) Proprietor: SUMITOMO CHEMICAL COMPANY, LIMITED
15 Kitahama 5-chome Higashi-ku
Osaka-shi Osaka-fu (JP)

(72) Inventor: Sunagawa, Makoto
No. 11-8-106 Sonehigashi-machi 2-chome
Toyonaka-shi, Osaka (JP)
Inventor: Matsumura, Haruki
No. 10-2-221 Sonehigashi-machi 2-chome
Toyonaka-shi, Osaka (JP)
Inventor: Inoue, Takaaki
No. 14-7, Mefu 2-chome
Takarazuka-shi Hyogo (JP)
Inventor: Hirohashi, Toshiyuki
No. 5-F-1107, Misawa-cho
Ibaraki-shi, Osaka (JP)

(74) Representative: Moore, Anthony John et al,
Gee & Co. Chancery House Chancery Lane
London WC2A 1QU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 023 097 B1

Courier Press, Leamington Spa, England.

(56) References cited:

JOURNAL OF THE AMERICAN CHEMICAL SOCIETY, Vol. 99, No. 7, 30 March 1977 W.F. HUFFMAN et al. "Nuclear Analogues of beta-Lactam Antibiotics. 1. The Total Synthesis of a 7-Oxo-1,3-Diazabicyclo (3.2.0) Heptane-2-Carboxylic Acid via a Versatile Monocyclic beta-Lactam Intermediate" pages 2352 to 2353

TETRAHEDRON LETTERS, No. 46, November 1978 Oxford S.D. SHARMA et al. "A New Method for the Synthesis of alpha-Amino-beta-Lactams" pages 4587 to 4590

J.F.W. Omie, Protective Groups in Organic Chemistry (Plenum Press 1973 ), 406-408

Houben-Weyl, Methoden der organischen Chemie Bd. XV/1 (Thieme-Verlag 1974), 714-720

**Description**

This invention relates to a process for de-arylmethylation of azetizinones (β-lactams) having a mono- or diarylmethyl group on the nitrogen atom of the β-lactam ring and to certain novel azetizinones among the products of the process which have anti-bacterial activity.

Oxidative cleavage using potassium persulfate is known as a method of removing a mono-arylmethyl group, e.g., a 2,4-dimethoxybenzyl group, substituted at the nitrogen atom of a β-lactam ring (See U.S. Patents 4072674 and 4166816, Tetrahedron Letters, 3c, 2771—74 (1979) and J.A.C.S., *99*, 2352 (1977)). However, this method has various disadvantages in operation and in yield: for example, it requires a relatively high reaction temperature; it cannot be applied to unstable compounds; it requires an oxidizing agent and thus the use of compounds having groups susceptible to oxidative conditions results in complicated products; and the reaction needs to be carried out in a buffering solution in order to prevent ring cleavage of the β-lactam ring.

We have found a method by which the reaction proceeds under milder conditions than with potassium persulfate; operation is simplified; and the desired product is obtained in higher yield.

The present invention provides a process for preparing β-lactam derivatives in which the nitrogen atom is unsubstituted (i.e. bears a hydrogen atom) and which are excellent synthetic intermediates for production of single β-lactam derivatives and bicyclic β-lactam derivatives useful as medicines having antibacterial activity, such as nocardicin A, thienamycin and their analogous compounds.

The invention provides a process of producing a β-lactam derivative of the general formula:

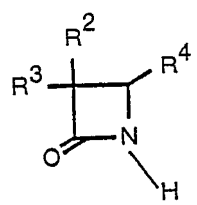

(X)

wherein

$R^2$ represents a hydrogen atom, an arylsulfonyl group, an amido group, an amino group, a $C_1$—$C_4$ alkoxycarbonylamino group, or a halo($C_1$—$C_3$) alkoxycarbonylamino group,

$R^3$ represents a hydrogen atom or a $C_1$—$C_4$ alkyl group, and

$R^4$ represents a $C_1$—$C_4$ alkoxycarbonyl group, a $C_2$—$C_4$ alkenyl group which may be substituted with an aryl group or a $C_1$—$C_4$ alkyl group which may be substituted with a $C_1$—$C_4$ alkylcarbonyl group or an arylcarbonyl group,

which process is characterised by reacting a β-lactam derivative having a mono- or diarylmethyl group on the nitrogen atom and being of the formula:

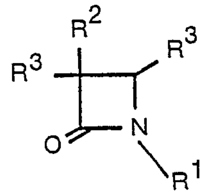

(Y)

wherein $R^2$, $R^3$ and $R^4$ are as defined above, and $R^1$ represents a mono- or diarylmethyl group, with ceric ammonium nitrate whereby the bond between the mono- or diarylmethyl $R^1$ group and the nitrogen atom is cleaved.

In the starting material, the mono- or diarylmethyl group ($R^1$) is suitably a methyl group substituted with one or two unsubstituted or substituted phenyl groups, such as 2,4-dimethoxybenzyl, di(4-methoxyphenyl)-methyl, 4-methoxybenzyl, phenylmethyl, diphenylmethyl, 3,4-dimethoxybenzyl or 4-dimethylaminobenzyl group, the 2,4-dimethoxybenzyl, 2,4,6-trimethoxybenzyl and di(4-methoxyphenyl)methyl groups being preferred.

Preferably the other substituents are as follows.

$R^2$ is a hydrogen atom or a phenylsulphonyl, amido, amino, $C_1$—$C_4$ alkoxycarbonylamino or 2,2,2-trichloroethoxycarbonylamino group;

$R^3$ is a hydrogen atom or an ethyl group; and,

$R^4$ is a $C_1$—$C_4$ alkoxycarbonyl group, an unsubstituted or substituted $C_2$—$C_4$ alkenyl group wherein the substituent is a phenyl group, or a substituted $C_1$—$C_4$ alkyl group wherein the substituent is a $C_1$—$C_4$ alkylcarbonyl or benzoyl group.

The cleavage reaction is preferably carried out in an inert solvent such as water; dimethylformamide; acetonitrile; alcohols such as methanol, ethanol or isopropanol; organic acids such as

acetic acid; and mixtures thereof: these solvents may also be used in combination with tetrohydrofuran, dioxane, benzene or toluene.

Ceric ammonium nitrate is generally used in an amount of about 2 to 3 mols per mol of the β-lactam derivative, and the reaction temperature is preferably between 0°C and 100°C although the reaction can be retarded or promoted by cooling or heating. Only the $R^1$ mono- or diarylmethyl group is cleaved, the $R^2$, $R^3$ and $R^4$ substituents remain unaltered. After completion of the reaction, the resulting product may be separated by conventional techniques used in organic synthesis.

Examples of the substituents comprised in $R^2$, $R^3$ and $R^4$ are as follows:

| | |
|---|---|
| alkyl | : methyl, ethyl, *n*-propyl, *n*-butyl. |
| alkoxycarbonyl | : methoxycarbonyl, ethoxycarbonyl, *n*-propyloxycarbonyl, *n*-butoxycarbonyl. |
| alkoxycarbonyl | : $C_2$—$C_4$ alkylcarbonyl, e.g. acetyl, propionyl. |
| arylcarbonyl | : unsubstituted or substituted benzoyl. |
| aryl | : unsubstituted or substituted phenyl. |
| arylsulfonyl | : unsubstituted or substituted phenylsulfonyl. |
| alkoxycarbonylamino | : methoxycarbonylamino, ethoxycarbonylamino. |
| halo alkoxycarbonylamino | : 2,2,2-trichloroethoxycarbonylamino. |
| amido | : unsubstituted or substituted alkanoylamino, e.g. acetylamino or phenoxyacetylamino, and arylcarbonylamino. |

Among the product compounds of formula (X) described in the Examples hereinbelow, those represented by the following formulae:

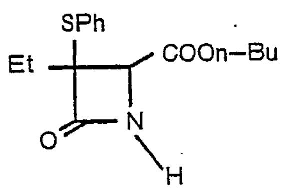

4-*n*-butoxycarbonyl-3-phenylthio-3-ethyl-2-azetizinone

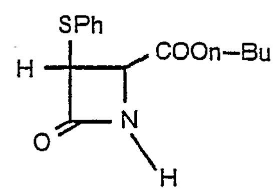

4-*n*-butoxycarbonyl-3-phenylthio-2-azetizinone

are novel and have antibacterial activity, particularly a strong antibacterial activity against gram-positive bacteria: these compounds are, therefore, particularly useful as medicines.

Starting materials (Y) wherein $R^2$ is amido may be prepared by the following method, which is also described and claimed in the EP-application 64797 divided herefrom.

An active acid anhydride derivative of a compound having the following formula (E):

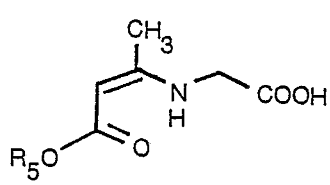

(E)

wherein $R_5$ represents a $C_1$—$C_4$ alkyl group such as methyl or ethyl, is reacted with a Schiff base having the formula (F):

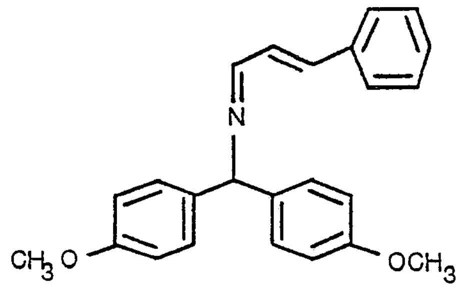

(F)

in the presence of a base in an inert solvent to produce a $\beta$-lactam having the following formula (G):

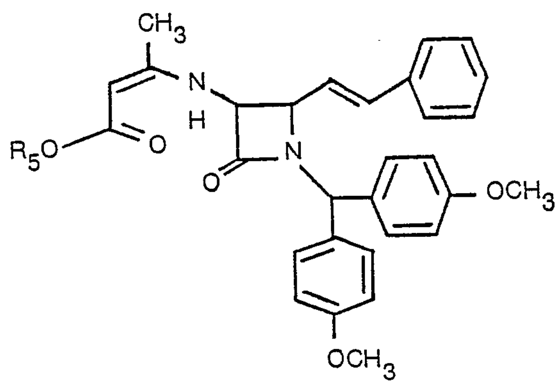

(G)

wherein $R_5$ is as defined above. The resultant crystalline $\beta$-lactam (G) is treated with an acid to produce compound (H), a derivative of compound (G) wherein the 3-position substituent on the azetizinone ring is $H_2N$-.

The compound (H) may be converted into various amido derivatives represented by the formula (Y') by reacting with an acylating agent such as trichloroethyl chloroformate or thienylacetyl chloride in the presence of a base, as illustrated by the following reaction scheme:

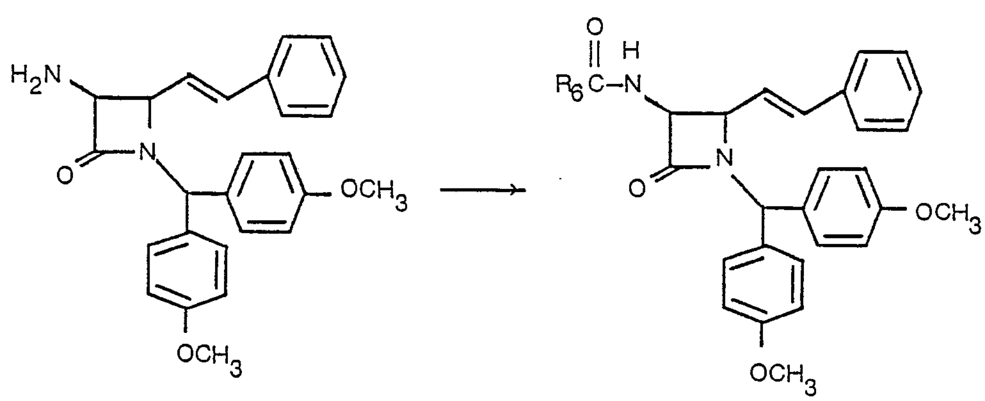

(H) (Y')

wherein $R_6$ represents, e.g.

$-OCH_2CCl_3$, 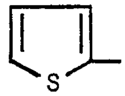 $CH_2-$, $PhOCH_2-$ or $CH_3$.

The amido derivatives of formula (Y) obtained by the process of the invention from the starting materials (Y′) shown above may be converted into the isocephalosporin derivative (B) of the following formula

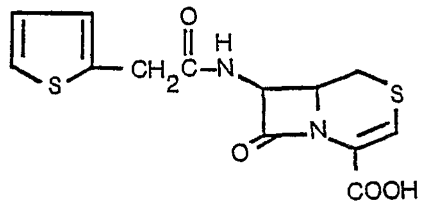

(B)

or the like by processes known in the literature.

The starting $\beta$-lactams (Y) described in the following Examples, other than the compounds (G) and (H), may be prepared by reacting an active acid anhydride of a carboxylic acid of the following formula:

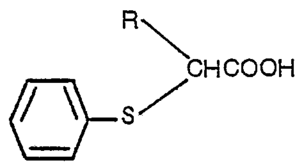

wherein R represents a hydrogen atom or an ethyl group, with a Schiff base represented by the following formula:

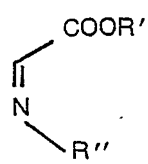

or

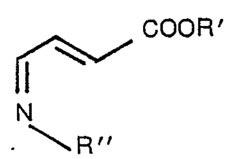

wherein R′ represents a $C_1$—$C_4$ alkyl or monoaryl-methyl group and R″ represents a mono- or diarylmethyl group, to produce a compound represented by the formula:

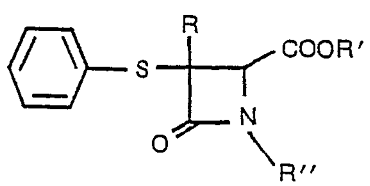

or

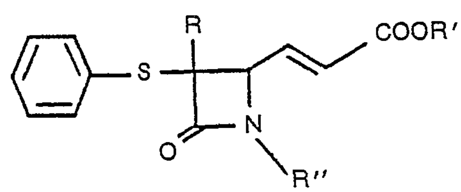

and, optionally, subjecting the resulting compound to a well known reaction, for example, reduction, oxidation or chain extension by Wittig reaction, under reaction conditions which satisfy the requirements of each of these reactions to produce the desired $\beta$-lactam. The preparation of these $\beta$-lactams used as starting material is illustrated in Examples 10 to 25.

The present invention will now be illustrated in greater detail by the following Examples. Unless otherwise indicated, all percentages and ratios are by weight.

Example 1

4-*n*-Butoxycarbonyl-3-benzenesulfonyl-3-ethyl-N-(2,4-dimethoxybenzyl)-2-azetizinone (74 mg) obtained in Example 18 in a mixture of acetic acid and water (1:1) (2 ml) was stirred with ceric ammonium nitrate (198 mg) at 100°C for 3 minutes, and the mixture was diluted with water and extracted with diethyl ether. The diethyl ether extract was washed with water, dried and evaporated to give an oil. The oil was chromatographed on silica gel to give 4-*n*-butoxycarbonyl-3-benzenesulfonyl-3-ethyl-2-azetizinone (86%).

$IR_{max}^{film}$ ($cm^{-1}$): 3300, 1782, 1742, 1210, 1145, 750, 730
NMR ($CDCl_3$): 0.93 (6H,t, J = 7Hz), 2.00 (2H, q, J = 7Hz), 4.18 (2H, t, J = 6Hz, 4.75 (1H, s), 6.78 (1H, br.s)

Example 2

4 - (2 - Phenylethenyl) - 3 - (2,2,2 - trichloroethoxycarbonyl)amino - N - di(*p* - anisyl)methyl - 2-azetizinone (59 mg) obtained in Example 12 in a mixture of dimethylformamide and water (9:1) (1.2 ml) was stirred with ceric ammonium nitrate (166 mg) at room temperature for 1 hour, and the mixture was diluted with ice-water and extracted with ethyl acetate. The ethyl acetate extract was washed with water, dried and evaporated to give an oil. The oil was chromatographed on silica gel to give 4-(2-phenylethenyl)-3-(2,2,2-trichloroethoxycarbonyl)amino-2-azetizinone (73%).
m.p. 157—161°C

$IR_{max}^{film}$ ($cm^{-1}$): 3340, 1750, 1615, 1518, 1257, 1180, 1100, 1048, 968

The following compounds were prepared by similar procedures to those in Examples 1 and 2.

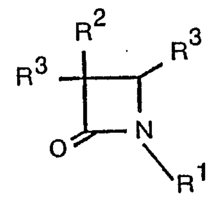

(Y)

| Ex. No. | Starting Material R1 | Starting Material R2 | Starting Material R3 | Starting Material R4 | Reaction Conditions Reagents | Reaction Conditions Temp. (Time) | Product R1 | Product Yield (%) | Product IR, NMR, etc. | Example No. for Preparation of Starting Material |
|---|---|---|---|---|---|---|---|---|---|---|
| 3 | DMB | $PhSO_2$ | Et | $-CH_2-CH_2-C(=O)-CH_3$ | CAN<br>$AcOH-H_2O$<br>(1:1) | 100°C<br>(1 hr) | H | 46 | ν: 3320, 1750, 1715, 1305, 1210, 1142, 1070, 1017<br>δ: 1.02 (3H, t J=7),<br>2.15 (3H, s)<br>2.58 (2H, t, J=6),<br>3.97 (1H, m) | 23 |
| 4 | DMB | H | Et | COOnBu | CAN<br>$AcOH-H_2O$<br>(1:1) | 100°C<br>(1 hr) | H | 75 | ν: 3270, 1760, 1285, 1205, 1150, 1100, 1038, 820<br>δ: 1.05 (3H, t, J=7),<br>3.47 (1H, m),<br>4.25 (2H, q, J=7),<br>6.37 (1H, br.s) | 24 |

| Ex. No. | Starting Material R₁ | Starting Material R₂ | Starting Material R₃ | Starting Material R₄ | Reaction Conditions Reagents | Reaction Conditions Temp. (Time) | Product R₁ | Product Yield (%) | Product IR, NMR, etc. | Example No. for Preparation of Starting Material |
|---|---|---|---|---|---|---|---|---|---|---|
| 5 | DAM | H | H | 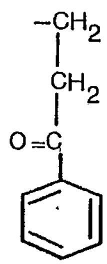 | CAN<br>DMF–$H_2O$ (9:1) | R.T. (1 hr) | H | 71 | $\nu$: 3270, 1740, 1680, 1355, 1252, 1208, 1175 | 22 |
| 6 | DAM | 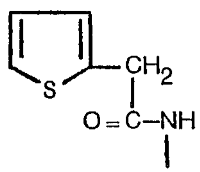 | H | 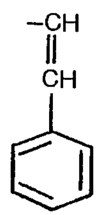 | CAN<br>DMF–$H_2O$ (9:1) | R.T. (1 hr) | H | 60 | m.p. (dec) 194–200°C<br>$\nu_{max}^{Nujol}$: 3420, 3250, 1772, 1662, 963 | 13 |
| 7 | DAM | 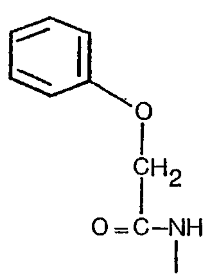 | H | 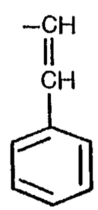 | CAN<br>DMF–$H_2O$ (9:1) | R.T. (1 hr) | H | 72 | m.p. 174–175°C<br>$\nu_{max}^{Nujol}$: 3270, 3210, 1770, 1725, 1672, 1242, 1225, 1082, 970 | 14 |

| Ex. No. | Starting Material R1 | Starting Material R2 | Starting Material R3 | Starting Material R4 | Reaction Conditions Reagents | Reaction Conditions Temp. (Time) | Product R1 | Product Yield (%) | Product IR, NMR, etc. | Example No. for Preparation of Starting Material |
|---|---|---|---|---|---|---|---|---|---|---|
| 8 | DAM | 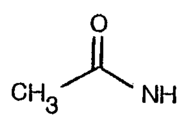 | H | 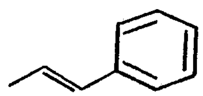 | CAN<br>DMF–$H_2O$<br>(9:1) | R.T.<br>(1 hr) | H | 22 | m.p. 213–214°C<br>$\nu_{max}^{Nujol}$ :<br>3275, 1768, 1715, 1650, 965 | 15 |
| 9 | DAM | $H_2N$– | H | 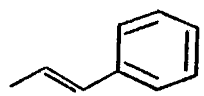 | CAN<br>DMF–$H_2O$<br>(9:1) | R.T.<br>(1.5 hr) | H | 85 | $\nu_{max}^{KBr}$ :<br>3375, 1755, 1718, 1490<br>$\delta$ (DMSO–$D_6$) :<br>4.21 (1H, d, J=3), 4.22 (1H, s), 6.38 (1H, dd, J=3, J=15), 6.58 (1H, d, J=15), 8.08 (1H, br. s) | 11 |

The following compounds may be prepared by procedures similar to those described in Examples 1 to 9.

4-(2-Phenylethenyl)-3-ethoxycarbonylamino-2-azetizinone
4-*n*-Butoxycarbonyl-2-azetizinone
4-Ethenyl-3-benzenesulfonyl-2-azetizinone

The symbols used in the tables have the meanings defined below.

DMB = 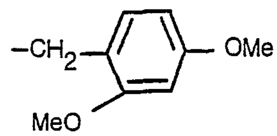 1)

DAM = 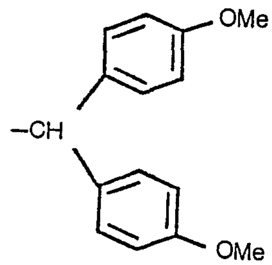 2)

3) $\nu = IR_{max}^{film}$ (cm$^{-1}$), unless otherwise indicated.
4) $\delta$ = NMR $\delta$ ($CDCl_3$), unless otherwise indicated.
5) nBu, Ac and Et indicate *n*-butyl, acetyl and ethyl groups, respectively
6) CAN = ceric ammonium nitrate
7) R.T. = room temperature

"Nujol" is a Registered Trade Mark for a liquid paraffin used as solvent in some IR spectral determinations.

The following Examples illustrate the preparation of starting materials used in the process of the invention.

Example 10

To N-(2-methoxycarbonyl-1-methylethenyl)glycine potassium salts (63.3 g) and triethylamine (30.3 g) in dry dichloromethane (1,250 ml) was added dropwise ethyl chloroformate (32.7 g) in dry dichloromethane (100 ml) at −20°C, and stirred for 30 minutes at the same temperature. To the reaction mixture was added dropwise trans-cinnamilidene-di(*p*-anisyl)methylamine (53.55 g) in dry dichloromethane (150 ml) at same temperature, and the reaction mixture was stood overnight at room temperature and filtered over the filter aid marketed under the registered Trade Mark "Celite". The filtrate was washed with aqueous sodium bicarbonate (4%) and then water, dried and evaporated in vacuo to give crude crystals, which were recrystallized from diisopropyl ether to give pure crystals of 4-(2-phenylethenyl)-3-(2-methoxycarbonyl-1-methylethenyl)amino-N-di(*p*-anisyl)methyl-2-azetizinone (76%). m.p. 164—166°C.

$IR_{max}^{Nujol}$ (cm$^{-1}$): 1737, 1650, 1606, 1457, 1370, 1263, 1155, 1025, 957, 827

The starting material, trans-cinnamylidene-di(*p*-anisyl)methylamine, was prepared from trans-cinnamaldehyde and di(*p*-anisyl)methylamine in a procedure similar to that described in Example 17.

Example 11

To 4-(2-phenylethenyl)-3-(2-methoxycarbonyl-1-methylethenyl)amino-N-di(*p*-anisyl)methyl-2-azetizinone (58.7 g) in dioxane (1,174 ml) were added *p*-toluenesulfonic acid monohydrate (23.96 g) and water (12.4 ml) and stood overnight at room temperature. The mixture was cooled with ice-water, and precipitates were collected by filtration and dried under reduced pressure to give a white powder of 4-(2-phenylethenyl)-3-amino-N-di(*p*-anisyl)methyl-2-azetizinone, *p*-toluenesulfonic acid salts. m.p. 172—174°C.

$IR_{max}^{Nujol}$ (cm$^{-1}$): 1760, 1610, 1508, 1460, 1373, 1245, 1175, 1030, 1003

Example 12

4-(2-phenylethenyl)-3-amino-N-di(*p*-anisyl)methyl-2-azetizinone, prepared from the corresponding *p*-toluenesulfonic acid salts (5.47 g) in the usual way, in dry dichloromethane (55 ml) and triethylamine (2.83 g) were added dropwise to 2,2,2-trichloroethyl chloroformate (5.94 g) in dry

dichloromethane (55 ml) at 5°C and stirred for 10 minutes. The reaction mixture was diluted with ice-water and extracted with dichloromethane. The dichloromethane extract was washed with water, dried and evaporated in vacuo to give an oily residue, which was crystallized from methanol to give pure crystals of 4-(2-phenylethenyl)-3-(2,2,2-trichloroethoxycarbonyl)amino-N-di(*p*-anisyl)methyl-2-azetizinone. m.p. 189—191°C.

$IR_{max}^{Nujol}$ ($cm^{-1}$): 1730, 1507, 1458, 1370, 1242, 1170, 1027

Example 13

To 4-(2-phenylethenyl)-3-amino-N-di(p-anisyl)methyl-2-azetizinone, prepared from the corresponding p-toluenesulfonic acid salts (207 mg), in dry dichloromethane (3 ml) and triethylamine (101 mg) was added dropwise 2-thienylacetyl chloride (120 mg) in dry dichloromethane (1 ml) at 5°C, followed by stirring for 1 hour. The reaction mixture was washed with water, 1N-hydrochloric acid, water, aqueous sodium bicarbonate and again water, dried and evaporated to give an oil. The oil was chromatographed on silica gel to give 4-(2-phenylethenyl)-3-(2-thienylacetyl)amino-N-di(*p*-anisyl)-methyl-2-azetizione.

$IR_{max}^{film}$ ($cm^{-1}$): 3270, 1740, 1650, 1245, 1177, 1030, 967, 830
NMR $\delta$ ($CDCl_3$): 3.63 (3H, s), 3.78 (3H, s), 4.39 (1H, dd, J = 5.5, J = 8.0), 5.31 (1H, dd, J = 5.5, J = 8.0), 5.70 (1H, dd, J = 8.0, J = 16.5), 5.87 (1H, s), 6.24 (1H, d, J = 16.5)

Example 14

4-(2-Phenylethenyl)-3-phenoxyacetylamino-N-di(p-anisyl)methyl-2-azetizinone was prepared by using phenoxyacetyl chloride instead of 2-thienylacetyl chloride in a procedure similar to that described in Example 13.

$IR_{max}^{film}$ ($cm^{-1}$): 3300, 1745, 1672, 1508, 1490, 1243, 1170, 1030, 962, 830
NMR $\delta$ ($CDCl_3$): 3.63 (3H, s), 3.77 (3H, s), 4.31 (2H, s), 4.42 (1H, dd, J = 5Hz, J = 8Hz), 5.40 (1H, dd, J = 5Hz, J = 8Hz), 5.85 (1H, dd, J = 8Hz, J = 16Hz), 5.97 (1H, s), 6.30 (1H, d, J = 16Hz)

Example 15

4-(2-Phenylethyl)-3-acetylamino-N-di(p-anisyl)methyl-2-azetizinone was also prepared by using acetyl chloride instead of 2-thienylacetyl chloride in a procedure similar to that described in Example 13.

$IR_{max}^{film}$ ($cm^{-1}$): 3280, 1745, 1655, 1610, 1245, 1172, 1030, 965
NMR ($CDCl_3$): 1.84 (3H, s), 3.64 (3H, s), 3.78 (3H, s), 4.41 (1H, dd, J = 5Hz, J = 8Hz), 5.35 (1H, dd, J = 5Hz, J = 8Hz)

Example 16

Di(*p*-anisyl)methylamine (20.0 g) and *n*-butyl glyoxylate (14.6 g) in dry toluene (1.2 l) were stirred at room temperature for 1 hour, azeotropically dehydrated with adding dry toluene, and cooled down to 65—70°C. Triethylamine (12.4 g) and then phenylthioacetyl chloride (18.4 g) in dry toluene (83 ml) were added dropwise at 65—70°C, followed by stirring 1 hour. The reaction mixture was washed with water, diluted hydrochloric acid, water, aqueous sodium bicarbonate, and again water, dried and evaporated in vacuo to give crude crystals, which were recrystallized from a mixture of diisopropyl ether and methanol (30:1) to give pure crystals of 4-*n*-butoxycarbonyl-3-phenylthio-N-di(*p*-anisyl)methyl-2-azetizinone. m.p. 90—91°C.

$IR_{max}^{film}$ ($cm^{-1}$): 1760, 1740, 1245, 1170, 1030, 820, 685

Example 17

2,4-Dimethoxybenzylamine (2.1 g) and *n*-butyl glyoxylate (1.82 g) in dry dichloromethane (200 ml) were stirred at room temperature for 1 hour, azeotropically dehydrated with adding dry dichloromethane, and cooled with ice-water. Triethylamine (2.24 g) and then $\alpha$-phenylthio-*n*-butyryl chloride (3.9 g) in dry dichloromethane were added dropwise at 0—5°C, followed by stirring at 0°C for 1 hour and then at room temperature for 2 hours. The reaction mixture was washed with water, diluted hydrochloric acid, aqueous potassium carbonate and again water, dried and evaporated to give an oil. The oil was chromatographed on silica gel to give 4-*n*-butoxycarbonyl-3-phenylthio-3-ethyl-N-(2,4-dimethoxybenzyl)-2-azetizinone.

$IR_{max}^{film}$ ($cm^{-1}$): 1770, 1740 (shoulder), 1290, 1210, 1155, 1035, 832, 750, 692
NMR ($CDCl_3$): 0.8—2.13 (12H, m), 3.63 (3H, s), 3.80 (3H, s), 6.33 (2H, m), 6.73 (1H, d, J = 8Hz)

Example 18

4-*n*-Butoxycarbonyl-3-phenylthio-3-ethyl-N-(2,4-dimethoxybenzyl)-2-azetizinone (1.6 g) and *m*-chloroperbenzoic acid (1.47 g) in chloroform (25 ml) were stirred overnight at room temperature, diluted with diethyl ether, washed with aqueous sodium bicarbnonate and then water, dried and evaporated in vacuo to give 4-*n*-butoxycarbonyl-3-benzenesulfonyl-3-ethyl-N-(2,4-dimethoxybenzyl)-2-azetizinone.

$IR^{film}_{max}$ ($cm^{-1}$): 1775, 1745, 1210, 1152, 1033, 760
NMR $\delta$ ($CDCl_3$): 1.00 (3H, t, J = 7Hz), 2.03 (2H, q, J = 7Hz), 3.75 (3H, s), 3.78 (3H, s), 4.12 (1H, d, J = 13Hz), 4.38 (1H, d, J = 13Hz), 6.36 (2H, m), 6.97 (1H, d, J = 8Hz)

Example 19

4-*n*-Butoxycarbonyl-3-phenylthio-N-di(*p*-anisyl)methyl-2-azetizinone (100 mg) in ethanol (5 ml) was refluxed with an excess of Raney nickel catalyst for 12 hours. The catalyst was removed by filtration. The filter aid was washed with ethanol. Washings and filtrate were combined and evaporated in vacuo to give 4-*n*-butoxycarbonyl-N-di(*p*-anisyl)methyl-2-azetizinone.

$IR^{film}_{max}$ ($cm^{-1}$): 1770, 1740, 1305, 1250, 1175, 1035, 825
NMR $\delta$ ($CDCl_3$): 0.73—1.73 (7H, m), 2.96 (1H, dd, J = 3.6Hz, J = 15Hz), 3.12 (1H, dd, J = 5.5Hz, J = 15Hz), 3.77 (6H, s), 3.95 (1H, dd, J = 3.6Hz, J = 5.5Hz), 5.83 (1H, s)

Example 20

4-*n*-Butoxycarbonyl-N-di(*p*-anisyl)methyl-2-azetizinone (3.2 g), sodium borohydride (0.93 g) and lithium iodide (3.26 g) in dry tetrahydrofuran were refluxed for 5 hours under nitrogen atmosphere, concentrated in vacuo, diluted with water, and extracted with ethyl acetate. The ethyl acetate extract was washed with water, dried, and evaporated. The residue was chromatographed on silica gel to give 4-hydroxymethyl-N-di(*p*-anisyl)methyl-2-azetizinone.

Example 21

To 4-hydroxymethyl-N-di(*p*-anisyl)methyl-2-azetizinone (0.78 g) in dry dimethyl sulfoxide (6.4 ml) were added dropwise triethylamine (2.34 g) and then sulfur trioxide pyridine complex (1.19 g) in dry dimethyl sulfoxide (6.4 ml) at room temperature, and the mixture was stirred for 1 hour, diluted with water and extracted with diethyl ether. The diethyl ether extract was washed with water, dilute hydrochloric acid and again water, dried and evaporated in vacuo to give 4-formyl-N-di(*p*-anisyl)methyl-2-azetizinone.

$IR^{film}_{max}$ ($cm^{-1}$): 1750, 1720 (shoulder), 1305, 1180, 1110, 1035, 960, 827, 817
NMR $\delta$ ($CDCl_3$): 3.76 (6H, s), 5.99 (1H, s), 9.07 (1H, d, J = 5.5Hz)

Example 22

4-Formyl-N-di(*p*-anisyl)methyl-2-azetizinone (0.75 g) and benzoylmethylene triphenylphosphoran (1.05 g) in dry tetrahydrofuran (20 ml) were refluxed for 2.5 hours and evaporated. The residue was chromatographed on silica gel to give 4-(2-benzoylethenyl)-N-di(*p*-anisyl)methyl-2-azetizinone.

This $\alpha,\beta$-unsaturated carbonyl derivative in ethanol (20 ml) was stirred with palladium on charcoal (10%) catalyst under hydrogen atomosphere at room temperature. The catalyst was removed by filtration over Celite. The filter aid was washed with ethanol. Washing and filtrate was combined and evaporated in vacuo to give 4-(2-benzoylethyl)-N-di(*p*-anisyl-methyl-2-azetizinone.

$IR^{film}_{max}$ ($cm^{-1}$): 1740, 1678, 1508, 1243, 1174, 1105, 1028, 807
NMR $\delta$ ($CDCl_3$): 1.83 (2H, q, J = 7Hz), 2.60—3.30 (4H), 3.75 (3H, s), 3.81 (3H, s), 5.74 (1H, s)

Example 23

4-(3-Oxobutyl)-3-benzenesulfonyl-3-ethyl-N-(2,4-dimethoxybenzyl)-2-azetizinone was prepared from 4-*n*-butoxycarbonyl-3-benzenesulfonyl-3-ethyl-N-(2,4-dimethoxybenzyl)-2-azetizinone by using acetylmethylenetriphenylphosphoran instead of benzoylmethylenetriphenylphosphoran in a procedure similar to those described in Examples 20, 21 and 22.

$IR^{film}_{max}$ ($cm^{-1}$): 1760, 1715, 1305, 1207, 1145, 1080, 1035, 938, 835
NMR $\delta$ ($CDCl_3$): 1.03 (3H, t, J = 7), 2.11 (3H, s), 3.79 (6H, s), 6.40 (2H, m), 7.02 (1H, d, J = 9)

Example 24

4-*n*-Butoxycarbonyl-3-phenylthio-3-ethyl-N-(2,4-dimethoxybenzyl)-2-azetizinone (100 mg) in ethanol (5 ml) was refluxed with an excess of Raney nickel catalyst for 12 hours. The catalyst was removed by filtration. The filter aid was washed with ethanol. Washings and filtrate were combined and

evaporated in vacuo to give 4-*n*-butoxycarbonyl-3-ethyl-N-(2,4-dimethoxybenzyl)-2-azetizinone.

$IR_{max}^{film}$ ($cm^{-1}$): 1760, 1740 (shoulder), 1295, 1212, 1158, 1035, 838
NMR ($CDCl_3$): 3.75 (3H, s), 3.78 (3H, s), 3.97 (1H, d, J = 6Hz), 4.10 (1H, d, J = 6Hz), 4.13 (1H, d, J = 15Hz), 4.60 (1H, d, J = 15Hz), 6.43 (2H, m), 7.10 (1H, d, J = 8Hz)

**Claims**

1. A process of producing a β-lactam derivative of the general formula:

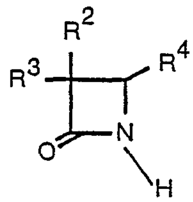

(X)

wherein
$R^2$ represents a hydrogen atom, an arylsulfonyl group, an amido group, an amino group, a $C_1$—$C_4$ alkoxycarbonylamino group, or a halo($C_1$—$C_3$) alkoxycarbonylamino group,
$R^3$ represents a hydrogen atom or a $C_1$—$C_4$ alkyl group, and
$R^4$ represents a $C_1$—$C_4$ alkoxycarbonyl group, a $C_2$—$C_4$ alkenyl group which may be substituted with an aryl group, or a $C_1$—$C_4$ alkyl group which may be substituted with a $C_1$—$C_4$ alkylcarbonyl group or an arylcarbonyl group.
which process is characterised by reacting a β-lactam derivative having a mono- or diarylmethyl group on the nitrogen atom and being of the formula:

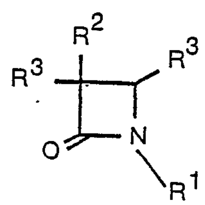

(Y)

wherein $R^2$, $R^3$ and $R^4$ are as defined above, and $R^1$ represents a mono- or diarylmethyl group, with ceric ammonium nitrate whereby the bond between the mono- or diarylmethyl $R^1$ group and the nitrogen atom is cleaved.

2. A process as claimed in Claim 1, wherein said starting derivative (Y) $R^1$ is a mono- or diphenylmethyl group in which each phenyl group may be unsubstituted or substituted.

3. A process as claimed in Claim 2, wherein $R^1$ is a 2,4-dimethoxybenzyl, 2,4,6-trimethoxybenzyl, di(4-methoxyphenyl)methyl 4-methoxybenzyl, phenylmethyl, diphenylmethyl, 3,4-dimethoxybenzyl or 4-dimethylaminobenzyl group.

4. A process as claimed in Claim 3, wherein in said starting derivative (Y) $R^1$ is a 2,4-dimethoxybenzyl or di(4-methoxyphenyl)methyl group; $R^2$ is a hydrogen atom or a phenylsulphonyl, amido, amino, $C_1$—$C_4$ alkoxycarbonylamino or 2,2,2-trichloroethoxycarbonylamino group; $R^3$ is a hydrogen atom or an ethyl group; and $R^4$ is a $C_1$—$C_4$ alkoxycarbonyl group, an unsubstituted or substituted $C_2$—$C_4$ alkenyl group wherein the substituent is a phenyl group, or a substituted $C_1$—$C_4$ alkyl group wherein the substituent is a $C_1$—$C_4$ alkylcarbonyl or benzoyl group.

5 A process as claimed in any of Claims 1 to 4, wherein the amido group for $R^2$ is an alkanoylamino or arylcarbonylamino group.

6. A process as claimed in any of Claims 1 to 5, which is carried out at a temperature of 0 to 100°C in an inert solvent.

7. A process as claimed in Claim 6, wherein the solvent comprises water.

8. 4-*n*-Butoxycarbonyl-3-phenylthio-3-ethyl-2-azetizinone.

9. 4-*n*-Butoxycarbonyl-3-phenylthio -2-azetizinone.

**Revendications**

1. Un procédé de préparation d'un dérivé de type β-lactame de formule générale:

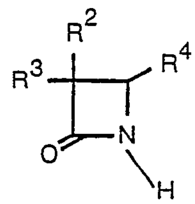

(X)

dans laquelle

$R^2$ représente un atome d'hydrogène, un groupe arylsulfonyle, un groupe amido, un groupe amino, un groupe alcoxy ($C_1$—$C_4$)carbonylamino ou un groupe halogénoalcoxy($C_1$—$C_3$)carbonylamino,

$R^3$ représente un atome d'hydrogène ou un groupe alkyle en $C_1$—$C_4$, et

$R^4$ représente un groupe alcoxy($C_1$—$C_4$) carbonyle, un groupe alcényle en $C_2$—$C_4$ pouvant être substitué par un groupe aryle ou un groupe alkyle en $C_1$—$C_4$ pouvant être substitué par un groupe alkyl($C_1$—$C_4$)carbonyle ou un groupe arylcarbonyle, lequel procédé est caractérisé par la réaction d'un dérivé de type β-lactame ayant un groupe mono- ou diarylméthyle sur l'atome d'azote et répondant à la formule:

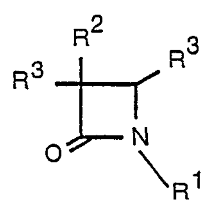

(Y)

dans laquelle $R^2$, $R^3$ et $R^4$ sont comme défini ci-dessus, et $R^1$ représente un groupe mono- ou diarylméthyle, avec du nitrate de cérium cérique et d'ammonium pour cliver la liaison entre le groupe mono- ou diarylméthyle $R^1$ et l'atome d'azote.

2. Un procédé comme revendiqué dans la revendication 1, dans lequel, dans ledit dérivé (Y) de départ, $R^1$ est un groupe mono- ou diphénylméthyle dont chaque groupe phényle peut être non substitué ou substitué.

3. Un procédé comme revendiqué dans la revendication 2, dans lequel $R^1$ est un groupe 2,4-diméthoxybenzyle, 2,4,6-triméthoxybenzyle, di(4-méthoxyphényl)méthyle, 4-méthoxybenzyle, phénylméthyle, diphénylméthyle, 3,4,-diméthoxybenzyle ou 4-diméthylaminobenzyle.

4. Un procédé comme revendiqué dans la revendication 3, dans lequel, dans ledit dérivé de départ (Y), $R^1$ est un groupe 2,4-diméthoxybenzyle ou di(4-méthoxyphényl)méthyle: $R^2$ est un atome d'hydrogène ou un groupe phénylsulfonyle, amido, amino, alcoxy($C_1$—$C_4$) carbonylamino ou 2,2,2-trichloroéthoxycarbonylamino; $R^3$ est un atome d'hydrogène ou un groupe éthyle; et $R^4$ est un groupe alcoxy($C_1$—$C_4$)carbonyle, un alcényle en $C_2$—$C_4$ non substitué ou substitué dont le substituant est un groupe phényle, ou un groupe alkyle en $C_1$—$C_4$ substitué dont le substituant est un groupe alkyl($C_1$—$C_4$) carbonyle ou benzoyle.

5. Un procédé comme revendiqué dans l'une quelconque des revendications 1 à 4, dans lequel le groupe amido $R^2$ est un groupe alcanoylamino ou arylcarbonylamino.

6. Un procédé comme revendiqué dans l'une quelconque des revendication 1 à 5, qui est réalisé à une température de 0 à 100°C dans un solvant inerte.

7. Un procédé comme revendiqué dans la revendication 6, dans lequel le solvant comprend de l'eau.

8. 4-n-butoxycarbonyl-3-phénylthio-3-éthyl-2-azétidinone.

9. 4-n-butoxycarbonyl-3-phénylthio-2-azétidinone.

**Patentansprüche**

1. Verfahren zur Herstellung eines β-Lactam-Derivats der allgemeinen Formel:

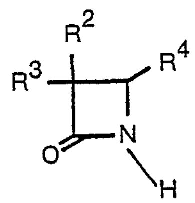

(X)

in der $R^2$ ein Wasserstoffatom, eine Arylsulfonylgruppe, eine Amidgruppe, eine Aminogruppe, eine $C_1$—$C_4$-Alkoxycarbonylaminogruppe oder eine Halogen-($C_1$—$C_3$)-alkoxycarbonylaminogruppe bedeutet,
$R^3$ ein Wasserstoffatom oder eine $C_1$—$C_4$-Alkylgruppe darstellt und
$R^4$ eine $C_1$—$C_4$-Alkoxycarbonylgruppe, eine $C_2$—$C_4$-Alkenylgruppe, die mit einem Arylrest substituiert sein kann, oder eine $C_1$—$C_4$-Alkylgruppe, die mit einer $C_1$—$C_4$-Alkylcarbonylgruppe oder einer Arylcarbonylgruppe substituiert sein kann,
dadurch gekennzeichnet, daß man ein β-Lactam-Derivat mit einer Mono- oder Diarylmethylgruppe am Stickstoffatom, das die nachstehende allgemeine Formel aufweist:

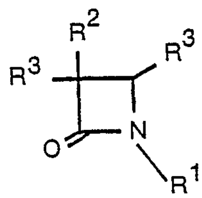

(Y)

in der $R^2$, $R^3$ und $R^4$ die vorstehende Bedeutung haben und $R^1$ eine Mono- oder Diarylmethylgruppe bedeutet, mit Cer(IV)-ammoniumnitrat umsetzt, wobei die Bindung zwischen der Mono- oder Diarylmethylgruppe $R^1$ und dem Stickstoffatom gespalten wird.

2. Verfahren nach Anspruch 1, dadurch gekennzeichnet, daß in dem genannten Ausgangsderivat (Y) der Rest $R^1$ eine Mono- oder Diphenylmethylgruppe bedeutet, in der jeder Phenylrest unsubstituiert oder substituiert sein kann.

3. Verfahren nach Anspruch 2, dadurch gekennzeichnet, daß $R^1$ eine 2,4-Dimethoxybenzylgruppe, 2,4,6-Trimethoxybenzylgruppe, Di-(4-methoxyphenyl)-methylgruppe, 4-Methoxybenzylgruppe, Phenylmethylgruppe, Diphenylmethylgruppe, 3,4-Dimethoxybenzylgruppe oder eine 4-Dimethylaminobenzylgruppe bedeutet.

4. Verfahren nach Anspruch 3, dadurch gekennzeichnet, daß in dem genannten Ausgangsderivat (Y) der Rest $R^1$ eine 2,4-Dimethoxybenzylgruppe oder Di-(4-methoxyphenyl)-methylgruppe bedeutet; $R^2$ ein Wasserstoffatom oder eine Phenylsulfonylgruppe, Amidgruppe, Aminogruppe, $C_1$—$C_4$-Alkoxycarbonylaminogruppe oder eine 2,2,2-Trichloräthoxycarbonylaminogruppe bedeutet; $R^3$ ein Wasserstoffatom oder eine Äthylgruppe darstellt und $R^4$ eine $C_1$—$C_4$-Alkoxycarbonylgruppe, einen unsubstituierten oder substituierten $C_2$—$C_4$-Alkenylrest, worin der Substituent eine Phenylgruppe ist, oder einen substituierten $C_1$—$C_4$-Alkylrest, worin der Substituent eine $C_1$—$C_4$-Alkylcarbonylgruppe oder eine Benzoylgruppe ist, darstellt.

5. Verfahren nach jedem der Ansprüche 1 bis 4, dadurch gekennzeichnet, daß der Amidrest im Substituenten $R^2$ ein Alkanoylamino- oder Arylcarbonylaminorest ist.

6. Verfahren nach jedem der Ansprüche 1 bis 5, dadurch gekennzeichnet, daß man die Umsetzungen bei Temperaturen von 0 bis 100°C in einem inerten Lösungsmittel durchführt.

7. Verfahren nach Anspruch 6, dadurch gekennzeichnet, daß das Lösungsmittel Wasser ist.

8. 4-n-Butoxycarbonyl-3-phenylthio-3-äthyl-2-azetizinon.

9. 4-n-Butoxycarbonyl-3-phenylthio-2-azetizinon.